# EUROPÄISCHE PATENTSCHRIFT

(11) **EP 2 524 666 B1**
(45) Veröffentlichungstag und Bekanntmachung des Hinweises auf die Patenterteilung: **10.05.2017**
(21) Anmeldenummer: 11166638.4
(22) Anmeldetag: 19.05.2011
(51) Int. Cl.: A61C 1/00, G01K 13/00, G01K 13/08, A61B 17/00

(54) **Medizinisches, insbesondere dentales, Handstück mit Temperaturmessvorrichtung**
Medical, in particular dental, handpiece with a temperature measuring device
Pièce à main médicale, en particulier dentaire, dotée d'un dispositif de mesure de température

(43) Veröffentlichungstag der Anmeldung: 21.11.2012
(60) Teilanmeldung: 17163464.5
(73) Patentinhaber: W & H Dentalwerk Bürmoos GmbH, 5111 Bürmoos (AT)
(72) Erfinder: Pruckner, Christian, 1190, Wien (AT)
(74) Vertreter: Benda, Ralf

(56) Entgegenhaltungen:
- WO-A1-2006/110670
- WO-A1-2009/072035
- GB-A- 2 438 877
- US-A- 5 123 839
- US-A1- 2009 259 244
- US-A1- 2009 322 541
- US-A1- 2010 055 642
- US-B2- 6 752 816

## Beschreibung

Die vorliegende Erfindung betrifft ein medizinisches, insbesondere dentales, Handstück mit einer Temperaturmessvorrichtung zur Messung der Temperatur des Handstücks oder eines Teils des Handstücks.

Ein derartiges Handstück ist zum Beispiel aus der Patentschrift US 6,786,897 B2 bekannt. Das Handstück ist mit einer lösbaren Kappe oder mit einem Anstrich oder Lack versehen, die einen temperatur-sensitiven oder thermochromen Farbstoff enthalten, der eine Temperaturänderung durch einen Farbwechsel anzeigt.

Der Nachteil dieses Handstücks besteht unter anderem darin, dass der thermochrome Farbstoff die Temperaturänderung nur unpräzise und verzögert wiedergibt und dass die Temperaturmessung ausschließlich an jenen Bauteilen des Handstücks erfolgt, an denen der thermochrome Farbstoff angebracht ist.

Die Anmeldeschrift US 2009/0322541 A1 zeigt ein Handstück, dessen Kopfgehäuse mit einem skelett- oder rippenartigen Gürtel mit einem zentralen Band und davon abstehenden seitlichen Detektionsbändern versehen ist, an dem unter anderem Temperatursensoren vorgesehen sind. Die seitlichen Detektionsbänder übertragen die von unterschiedlichen Wärmequellen abgegebene Wärme mittels Wärmeleitung zu den Temperatursensoren.

Der Nachteil dieses Handstücks besteht unter anderem wiederum in der aufgrund der Wärmeleitung über die Detektionsbänder verzögerten und unpräzisen Temperaturmessung sowie in der durch die Anordnung der Detektionsbänder auf bestimmte Regionen oder Bauteile des Handstücks beschränkten Temperaturmessung.

Der vorliegenden Erfindung liegt daher die Aufgabe zugrunde ein medizinisches, insbesondere dentales, Handstück mit einer alternativen Temperaturmessvorrichtung zur Messung der Temperatur des Handstücks oder eines Teils des Handstücks zu schaffen, welches insbesondere einen oder mehrere der oben genannten Nachteile nicht aufweist.

Diese Aufgabe ist durch ein medizinisches, insbesondere dentales, Handstück gemäß dem Anspruch 1 gelöst.

Gemäß einem Ausführungsbeispiel sind mehrere Wärmequellen am oder im Handstück vorhanden, wobei insbesondere eine Wärmequelle das Handstück oder Teile des Handstücks stärker erwärmt als eine andere Wärmequelle oder wobei insbesondere eine Wärmequelle mehr Wärme oder Wärmestrahlung emittiert als eine andere Wärmequelle. Beispielsweise sind im Handstück oder im Handstückkopf zwei Lager, insbesondere Wälzlager, vorgesehen, wobei ein erstes Lager aufgrund eines Gebrechens oder aufgrund einer Verschmutzung wärmer wird als ein zweites Lager.

Selbstverständlich können die im Vorstehenden genannten Wärmequellen auch andere Bauteile des Handstücks als die genannten Lager umfassen, insbesondere jegliche relativ zueinander bewegte Bauteile des Handstücks, vorzugsweise bewegbare und stillstehende, einander kontaktierende Bauteile, zum Beispiel einen Werkzeughalter und eine Werkzeuglösevorrichtung des Handstücks oder Teile des Werkzeughalters und der Werkzeuglösevorrichtung oder einen Rotor eines Motors oder eines Generators oder die Außenhülse des Handstücks und ein relativ dazu bewegliches Bauteil des Handstücks. Eine Wärmequelle kann auch durch ein mit elektrischer Energie versorgbares Bauteil, wie zum Beispiel eine Beleuchtungsvorrichtung, einen Elektromotor, insbesondere dessen Spulen, eine elektrische oder elektronische Schaltung, einen Sensor oder Detektor etc. gebildet sein. Diese Aufzählung der möglichen Wärmequellen ist beispielhaft und nicht vollständig und trifft in entsprechender Weise auf alle weiteren beschriebenen Ausführungsbeispiele zu.

Ein Vorteil dieses Handstücks besteht darin, dass die Temperaturmessvorrichtung somit nicht ausschließlich oder nicht vorwiegend die Temperatur eines bestimmten Bauteils misst, sondern dass mittels der Temperaturmessvorrichtung ein gemittelter Temperaturwert oder Durchschnittstemperaturwert ermittelbar ist. Somit können in vorteilhafter Weise mehrere Wärmequellen durch eine einzige Temperaturmessvorrichtung überwacht werden. Die mehreren Wärmequellen geben zumindest einen Teil ihrer Wärme in den Innenraum des Handstücks ab, bevorzugt in ein Teilvolumen des Handstücks, insbesondere in den Handstückkopf des Handstücks, in welchem ein Werkzeughalter für das mit dem Handstück verbindbare Werkzeug vorgesehen ist. Das im Vorstehenden genannte Teilvolumen bzw. der Innenraum, dessen gemittelte Temperatur oder Durchschnittstemperatur messbar ist, ist insbesondere größer als das Volumen der Temperaturmessvorrichtung oder des Sensors der Temperaturmessvorrichtung, vorzugsweise mehrfach größer als das Volumen der Temperaturmessvorrichtung oder des Sensors der Temperaturmessvorrichtung.

Der Innenraum des Handstücks ist vorzugsweise durch ein Fluid, insbesondere Luft, enthaltendes Volumen gebildet, welches Bauteile des Handstücks voneinander trennt, wobei in dem Volumen die Temperaturmessvorrichtung vorgesehen ist. In dieses Volumen gibt die zumindest eine Wärmequelle ihre Wärme ab. Alternativ oder zusätzlich ist die Temperaturmessvorrichtung räumlich von der zumindest einen Wärmequelle beabstandet. Beide Maßnahmen erhöhen die Unabhängigkeit der Temperaturmessung von der zumindest einen Wärmequelle oder erleichtern die Bestimmung eines gemittelten Temperaturwerts oder Durchschnittstemperaturwerts.

Erfindungsgemäß ist ein medizinisches, insbesondere dentales, Handstück vorgesehen, das umfasst: Eine Außenhülse, eine Antriebsvorrichtung zum in Bewegung Versetzen eines mit dem Handstück verbindbaren Werkzeugs und eine Temperaturmessvorrichtung zur Messung der Temperatur des Handstücks oder eines Teils des Handstücks, wobei die Temperaturmessvorrichtung als elektrische Temperaturmessvorrichtung ausgebildet ist.

Ein Vorteil einer elektrischen Temperaturmessvorrichtung besteht unter anderem in der präzisen und raschen Ermittlung des Temperarturwerts.

Erfindungsgemäß umfasst die elektrische Temperaturmessvorrichtung ein Material, dessen elektrischer Widerstand oder dynamischer Widerstand in Abhängigkeit der Temperatur variabel ist. Das Material umfasst zum Beispiel einen Ohmschen Widerstand, einen elektrischen Leiter, insbesondere ein Metall, eine Keramik (ein gesintertes Metalloxid) oder einen dotierten Halbleiter. Bevorzugt ist zumindest eines dieser Materialien Teil eines Sensors der elektrischen Temperaturmessvorrichtung. Je nach Ausführung ist ein derartiger Sensor als aktiver Sensor, der zum Bestimmen der Temperatur eine Strom- oder Spannungsversorgung benötigt, oder als passiver Sensor ausgebildet, der zum Bestimmen der Temperatur keine Strom- oder Spannungsversorgung benötigt.

Der Begriff Handstück umfasst insbesondere mit einer Hand greifbare medizinische oder dentale Geräte, zum Beispiel gerade, pistolenförmige, gewinkelte oder gebogene Handgriffe, die im Dentalbereich oftmals als Winkelstücke bezeichnet werden, Teile von Handstücken oder Handgriffen, insbesondere einen Kopfabschnitt, der zum Beispiel mit einem davon lösbaren Griffabschnitten verbindbar ist, sowie Kupplungen, Adapter, Verbindungsstücke und Antriebseinheiten, zum Beispiel elektrische oder pneumatische Motoren. Unter der Bezeichnung Handstück werden des Weiteren sowohl schnurlose Handstück verstanden, insbesondere mit einer austauschbaren oder aufladbaren Energiequelle, als auch Handstück, die eine Versorgungsleitung und eine damit verbundene Steuer-, Regel- und / oder Versorgungseinheit umfassen. Gemäß einem bevorzugten Ausführungsbeispiel, welches auf alle genannten Ausführungsformen und -beispiele anwendbar ist, weist das Handstück einen Handstückkopf auf, in welchem ein Werkzeughalter für das mit dem Handstück verbindbare Werkzeug und die Temperaturmessvorrichtung, und der Temperatursensor der Temperaturmessvorrichtung, vorgesehen sind.

Die Erfindung wird nachfolgend anhand bevorzugter Ausführungsbeispiele und Bezug nehmend auf die beigefügten Zeichnungen erläutert:
Figur 1 zeigt eine Außenansicht eines Ausführungsbeispiels eines erfindungsgemäßen Handstücks mit einer Temperaturmessvorrichtung zur Messung der Temperatur des Handstücks oder eines Teils des Handstücks.
Figur 2 zeigt ein Beispiel eines Handstückkopfs mit einer Temperaturmessvorrichtung zur Messung der Temperatur des Handstücks oder eines Teils des Handstücks, wobei die Temperaturmessvorrichtung ein Pyrometer oder einen Infrarotsensor umfasst, die nicht Teil der Erfindung sind.
Die Figuren 3 - 5 zeigen Ausführungsbeispiele von Handstückköpfen mit je einer Temperaturmessvorrichtung zur Messung der Temperatur des Handstücks oder eines Teils des Handstücks, wobei die elektrische Temperaturmessvorrichtung ein Material umfasst, dessen elektrischer Widerstand oder dynamischer Widerstand in Abhängigkeit der Temperatur variabel ist.

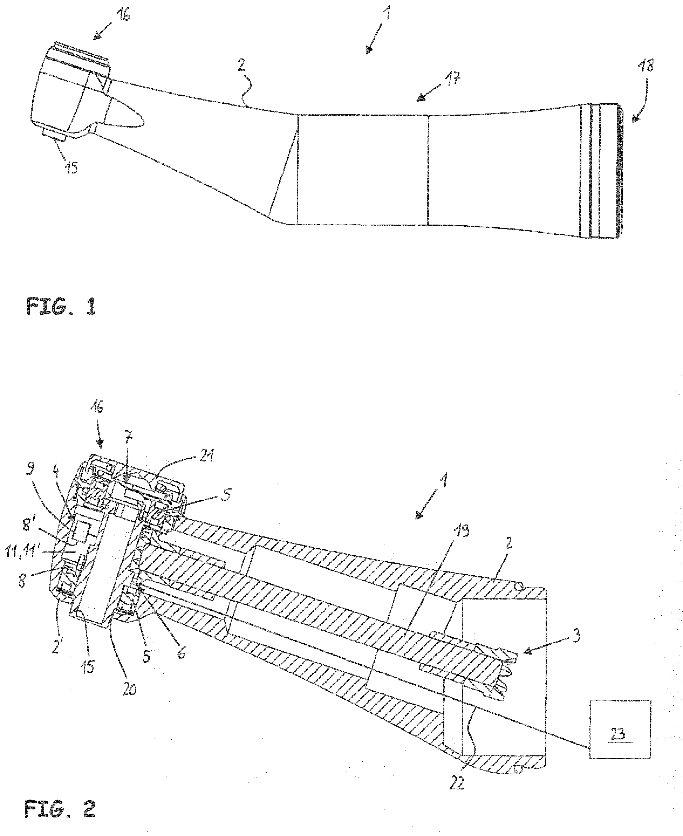

Die Figur 1 zeigt eine Außenansicht eines gebogenen Handstücks 1, welches oftmals als Winkelstück bezeichnet wird, mit seiner Außenhülse 2. Das Handstück 1 weist einen, insbesondere gebogenen, Griffteil 17 und einen Kopfteil 16 auf, in welchem ein Werkzeughalter 15 zum lösbaren Verbinden oder Aufnehmen eines mit dem Handstück 1 verbindbaren Werkzeugs angeordnet ist. Der Werkzeughalter 15 ist vorzugsweise beweglich im Handstück 1 angeordnet, zum Beispiel drehbar, wobei die Drehbarkeit insbesondere durch Lager 5, vorzugsweise Wälz- oder Kugellager, gewährleistet ist (siehe Figur 2).

An dem dem Kopfteil 16 gegenüber liegenden Ende des Handstücks 1 ist eine Kupplungs- oder Anschlussvorrichtung 18 vorgesehen, welche ausgebildet ist, das Handstück 1 mit einer oder mehreren Fluidquellen, zum Beispiel einer Flüssigkeitsquelle, insbesondere Wasser, oder einer Gasquelle, insbesondere Druckluft, zu verbinden. Vorzugsweise ist das Handstück 1 über die Anschlussvorrichtung 18 auch mit einer elektrischen Spannungsquelle und / oder einer Antriebsvorrichtung, zum Beispiel einen Elektromotor oder einem pneumatisch betreibbaren Motor, verbindbar. Wahlweise sind weitere elektrische Leitungen oder Kontakte zur Daten- und / oder Signalübertragung an der Kupplungsvorrichtung 18 vorgesehen.

Durch das Handstück 1 bzw. die Außenhülse 2 erstrecken sich eine oder mehrere Fluidleitungen von der Anschlussvorrichtung 18 in Richtung des Kopfteils 16 oder bis in den Kopfteil 16. Gegebenenfalls weist das Handstück 1 auch eine oder mehrere Wellen 19 auf, vorzugsweise mittels Zahnrädern oder Getrieben 6 verbunden, die als Antriebsvorrichtung 3 dienen und eine Antriebsbewegung auf den Werkzeughalter 15 übertragen (siehe Figur 2).

Die Figur 2 zeigt einen Schnitt durch einen Teil des Griffteils 17 und insbesondere durch den Kopfteil 16 des Handstücks 1. Der Kopfteil 16 weist eine hohle Kopfhülse 2' auf, die einen Innenraum 11 bildet. Der Innenraum 11 ist insbesondere als Fluid, insbesondere Luft, enthaltendes Volumen 11' ausgebildet, in dem unterschiedliche Bauteile des Kopfteils aufgenommen sind, zum Beispiel in Bezug auf die Hülse 2, 2' stationäre Bauteile oder in Bezug auf die Hülse 2, 2' bewegliche Bauteile, insbesondere die Lager 5, der Werkzeughalter 15, zumindest Teile eines Getriebes 6 oder von Zahnrädern, welche die Antriebsbewegung der Antriebsvorrichtung 3 von der Welle 19 auf den Werkzeughalter 15 übertragen, zumindest Teile eine Vorrichtung 7 zum Lösen des Werkzeugs aus dem Werkzeughalter oder weitere Bauteile wie Federelemente, Stützelemente, etc. An einer Seite des Kopfteils 16 ist des Weiteren eine Öffnung 20 vorgesehen, durch welche das Werkzeug in den Kopfteil 16 oder in den Werkzeughalter 15 einsetzbar ist bzw. daraus entnehmbar ist. An der Seite des Kopfteils 16, die der Öffnung 20 gegenüber liegt, ist ein Betätigungselement 21 zum Betätigen der Werkzeuglösevorrichtung 7 vorgesehen, insbesondere ein Druckelement oder Druckdeckel.

Zumindest einige der im Vorstehenden genannten Bauteile oder Teile davon können als Wärmequellen ausgebildet sein, zum Beispiel die Lager 5, das Getriebe 6 oder die Werkzeuglösevorrichtung 7. Selbstverständlich können jedoch auch andere Bauteile des Handstücks Wärmequellen bilden, insbesondere ein elektromagnetische Strahlung emittierendes Bauteil, zum Beispiel eine Beleuchtungsvorrichtung, ein Heizelement für Medien, ein elektrisches oder elektronisches Bauteil, etc. Gemeinsam ist allen diesen Wärmequellen, dass sie Wärme in das Innere des Handstücks 1 und / oder an die Außenhülse 2, 2' des Handstücks abgeben. Um die die Temperatur des Handstücks 1 oder eines Teils des Handstücks 1, vorzugsweise der Außenhülse 2, 2', oder des Innenraums 11 des Handstücks 1 zu bestimmen und insbesondere eine zu starke Erwärmung zu verhindern, ist deshalb am oder im Handstück 1, insbesondere am oder im Kopfteil 16, eine Temperaturmessvorrichtung 4 vorgesehen.

Über eine elektrische Leitung oder eine Signalleitung 22 ist die Temperaturmessvorrichtung 4 mit einer Einheit 23 verbunden, die wahlweise als Auswerteeinheit zum Auswerten der Messsignale der Temperaturmessvorrichtung 4 und / oder als Regel- oder Steuereinheit zum temperaturabhängigen Regeln oder Steuern des Betriebs des Handstücks 1, zum Beispiel zum Unterbrechen des Betriebs des Handstücks 1 bei Erreichen oder Überschreiten eines vorbestimmten Temperaturgrenzwerts, und / oder als Anzeigeeinheit zum Anzeigen des gemessenen oder bestimmten Temperaturwerts oder eines Temperaturgrenzwerts ausgebildet ist. Vorzugsweise sind zumindest Teile der Einheit 23 am oder im Handstück 23 vorgesehen. Gemäß einem besonders bevorzugten Ausführungsbeispiel ist die gesamte Temperaturmessvorrichtung 4 einschließlich der Leitung 22 und der Einheit 23 im oder am Handstück 1 angeordnet, wodurch insbesondere ein in Bezug auf die Temperaturmessung, -auswertung und die davon abhängige Steuerung oder Regelung unabhängiges Handstück 1 entsteht.

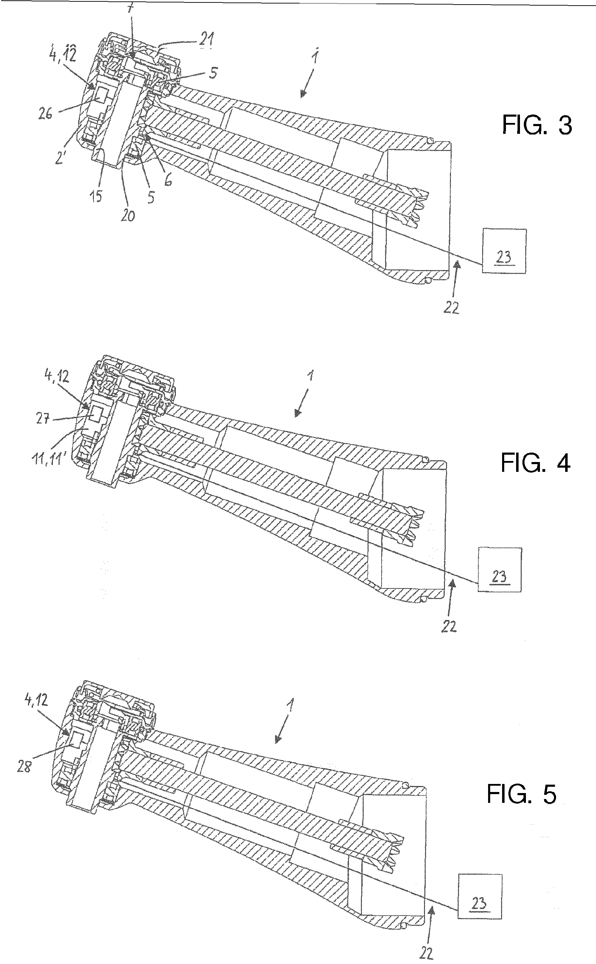

Die im Vorstehenden unter Bezug auf die Figuren 1 und 2 beschriebenen Merkmale gelten für alle in den Figuren 3 - 5 beschriebenen Ausführungsbeispiele. Im Folgenden werden nun insbesondere jene Merkmale beschrieben, in denen sich die Handstücke 1 der Figuren 3 - 5 unterscheiden, bevorzugt die unterschiedlichen Temperaturmessvorrichtungen 4.

Die Figuren 3 - 5 zeigen Handstücke 1 mit den elektrischen Temperaturmessvorrichtungen 12, wobei jede dieser Temperaturmessvorrichtungen 12 ein Material umfasst, dessen elektrischer Widerstand oder dynamischer Widerstand in Abhängigkeit der Temperatur variabel ist.

Die Temperaturmessvorrichtungen 4, 12 der Figuren 3 - 5 sind derart in den Handstücken 1 angeordnet und ausgebildet, dass sie Wärme oder Wärmestrahlung mehrerer in der Außenhülse 2 des Handstücks 1 angeordneter Wärmequellen (zum Beispiel der Lager 5, des Getriebes 6 und Teile der Werkzeuglösevorrichtung 7) empfangen, um einen gemittelten Temperaturwert der mehreren Wärmequellen 5, 6, 7 zu bestimmen. Um die Wärme oder Wärmestrahlung der mehreren Wärmequellen 5, 6, 7 zu empfangen, ist die Temperaturmessvorrichtung 4 zwischen den Wärmequellen 5, 6, 7 angeordnet und optional zumindest eines der folgenden Merkmale verwirklicht: Die Temperaturmessvorrichtung 4, 12 ist im Innenraum 11, 11' des Handstücks 1, beabstandet von den Wärmequellen 5, 6, 7 angeordnet; die Wärmequellen 5, 6, 7 umgeben die Temperaturmessvorrichtung 4, 12; die Wärme emittierenden Oberflächen der Wärmequellen 5, 6, 7 sind räumlich von einer die Wärme der Wärmequelle 5, 6, 7 empfangenden Oberfläche der Temperaturmessvorrichtung 4, 12 beabstandet; die Temperaturmessvorrichtung 4, 12 weist mehrere Wärme der Wärmequelle 5, 6, 7 empfangende Oberflächen 8' auf, die insbesondere in unterschiedliche Richtungen weisen.

Optional können die Temperaturmessvorrichtungen 4, 12 der Figuren 3 - 5 ausgebildet und / oder angeordnet, die Temperatur des durch die zumindest eine Wärmequelle 5, 6, 7 erwärmbaren Innenraums 11 des Handstücks 1, bevorzugt die Temperatur des im Innenraum 11 oder Volumen 11' enthaltenden Fluids, zu messen.

Vorzugsweise ist die Temperaturmessvorrichtung 4, 12, insbesondere der Sensor der Temperaturmessvorrichtung 4, 12, zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet (siehe Figuren 2 und 4). Alternativ ist die Temperaturmessvorrichtung 4, 12, insbesondere der Sensor der Temperaturmessvorrichtung 4, 12, im Bereich der Werkzeuglösevorrichtung 7, insbesondere zwischen dem dem Betätigungselement 21 näher gelegenen Lager 5 und dem Betätigungselement 21 angeordnet. Alternativ ist die Temperaturmessvorrichtung 4, 12, insbesondere der Sensor der Temperaturmessvorrichtung 4, 12, in der Nähe jenes Lagers 5, das der Werkzeugöffnung 20 näher liegt, angeordnet.

Die Temperaturmessvorrichtung 4, 12 des Handstücks 1 der Figur 3 weist einen Messwiderstand 26 auf, der vorzugsweise aus einem Edelmetall besteht, dessen elektrischer Widerstand (ohmsche Widerstand) in Abhängigkeit der Temperatur, insbesondere der Temperatur des den Widerstand 26 umgebenden Fluids des Innenraums 11, 11', variabel ist. Eine in der Einheit 23 vorgesehene Strom- oder Spannungsquelle bildet mit der elektrischen Leitung 22 und dem Messwiderstand 26 einen elektrischen Stromkreis. Die Veränderung des von der Strom- oder Spannungsquelle abgegebenen, über den Messwiderstand 26 geleiteten elektrischen Messsignals, insbesondere der Spannungsabfall eines als Messsignal dienenden konstanten Messstroms, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Temperaturmessvorrichtung 4, 12 des Handstücks 1 der Figur 4 weist einen Halbleitersensor 27 auf, vorzugsweise eine Halbleiterdiode, insbesondere eine dotierte Siliziumdiode, dessen dynamischer Widerstand (Schleusenspannung an einem p-n-Übergang oder einem Metall-Halbleiter-Übergang des Halbleitersensors 27) in Abhängigkeit der Temperatur, insbesondere der Temperatur des den Halbleitersensor 27 umgebenden Fluids des Innenraums 11, 11', variabel ist. Eine in der Einheit 23 vorgesehene Strom- oder Spannungsquelle bildet mit der elektrischen Leitung 22 und dem Halbleitersensor 27 einen elektrischen Stromkreis. Die Veränderung des von der Strom- oder Spannungsquelle abgegebenen, über den Halbleitersensor 27 geleiteten elektrischen Messsignals, insbesondere der Spannungsabfall eines als Messsignal dienenden konstanten Messstroms, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Temperaturmessvorrichtung 4, 12 des Handstücks 1 der Figur 5 weist einen metallischen Widerstand 28 auf, wobei die Spannung des thermischen Widerstandsrauschens (d.h. die Dichteschwankungen der Transportelektronen des Widerstands 28 aufgrund ihrer thermischen Bewegung) in Abhängigkeit der Temperatur, insbesondere der Temperatur des den Widerstand 28 umgebenden Fluids des Innenraums 11, 11', variabel ist. Eine in der Einheit 23 vorgesehene Strom- oder Spannungsquelle bildet mit der elektrischen Leitung 22 und dem Widerstand 28 einen elektrischen Stromkreis. Die Veränderung des von der Strom- oder Spannungsquelle abgegebenen, über den Widerstand 28 geleiteten elektrischen Messsignals, insbesondere die Veränderung der elektrischen Spannung, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

## Patentansprüche

1. Medizinisches, insbesondere dentales, Handstück (1), umfassend: Ein Griffteil (17) und ein Kopfteil (16), in welchem ein Werkzeughalter (15) zum lösbaren Verbinden oder Aufnehmen eines mit dem Handstück (1) verbindbaren Werkzeugs angeordnet ist, eine Außenhülse (2), wobei das Kopfteil (16) eine hohle Kopfhülse (2') aufweist, die einen Innenraum (11) bildet, eine Antriebsvorrichtung (3) zum in Bewegung Versetzen eines mit dem Handstück (1) verbindbaren Werkzeugs, in Bezug auf die Kopfhülse (2') bewegliche Bauteile, die in dem Kopfteil (16) aufgenommen sind, insbesondere Lager (5), der Werkzeughalter (15), zumindest Teile eines Getriebes (6) oder von Zahnrädern, welche die Antriebsbewegung der Antriebsvorrichtung (3) auf den Werkzeughalter (15) übertragen, oder zumindest Teile eine Vorrichtung (7) zum Lösen des Werkzeugs aus dem Werkzeughalter (15), und eine Temperaturmessvorrichtung (4) zur Messung der Temperatur des Handstücks (1) oder eines Teils des Handstücks (1), wobei zumindest einige der in dem Kopfteil (16) aufgenommenen, in Bezug auf die Kopfhülse (2') beweglichen Bauteile (5, 6, 7, 15) oder Teile davon Wärmequellen bilden, welche Wärme in das Innere des Handstücks (1) und / oder an die Kopfhülse (2') abgeben, wobei
die Temperaturmessvorrichtung (4) als elektrische Temperaturmessvorrichtung (12) ausgebildet ist, wobei die elektrische Temperaturmessvorrichtung (12) einen Temperatursensor aufweist, der in dem Kopfteil (16) des Handstücks (1) angeordnet ist und Wärme oder Wärmestrahlung mehrerer in der Außen- oder Kopfhülse (2, 2') des Handstücks (1) angeordneter Wärmequellen (5, 6, 7) empfängt, wobei zumindest eine dieser mehreren Wärmequellen ein bewegliches Bauteil (5, 6, 7, 15) aufweist, und einen gemittelten Temperaturwert oder Durchschnittstemperaturwert des Handstücks (1) oder eines Teils des Handstücks (1) oder eines Innenraums (11) des Handstücks (1) misst, der aus den Wärmeemissionen der mehreren Wärmequellen (5, 6, 7) resultiert,
die elektrische Temperaturmessvorrichtung (12) ein Material umfasst, dessen elektrischer Widerstand oder dynamischer Widerstand in Abhängigkeit der Temperatur variabel ist, und
die Temperaturmessvorrichtung (12) zwischen den Wärmequellen (5, 6, 7) angeordnet ist.

2. Medizinisches, insbesondere dentales, Handstück (1), nach Anspruch 1, **dadurch gekennzeichnet, dass**
die Temperaturmessvorrichtung (12) im Innenraum (11, 11') des Handstücks (1), beabstandet von den Wärmequellen (5, 6, 7) angeordnet ist oder dass die Wärmequellen (5, 6, 7) die Temperaturmessvorrichtung (12) umgeben oder dass die Wärme emittierenden Oberflächen der Wärmequellen (5, 6, 7) räumlich von einer die Wärme der Wärmequelle (5, 6, 7) empfangenden Oberfläche der Temperaturmessvorrichtung (12) beabstandet sind oder dass die Temperaturmessvorrichtung (12) mehrere Wärme der Wärmequelle (5, 6, 7) empfangende Oberflächen (8') aufweist, die insbesondere in unterschiedliche Richtungen weisen.

3. Medizinisches, insbesondere dentales, Handstück (1), nach einem der vorstehenden Ansprüche, **dadurch gekennzeichnet, dass**
die elektrische Temperaturmessvorrichtung (12) einen Halbleitersensor (27), vorzugsweise eine Halbleiterdiode, oder einen Sensor mit einem Ohmschen Widerstand, einem elektrischen, insbesondere metallischen, Leiter, einer Keramik, insbesondere einem gesinterten Metalloxid, oder einem dotierten Halbleiter aufweist.

4. Medizinisches, insbesondere dentales, Handstück (1), nach einem der vorstehenden Ansprüche, **dadurch gekennzeichnet, dass**
der Sensor als aktiver Sensor, der zum Bestimmen der Temperatur eine Strom- oder Spannungsversorgung benötigt, oder als passiver Sensor, der zum Bestimmen der Temperatur keine Strom- oder Spannungsversorgung benötigt, ausgebildet ist.

5. Medizinisches, insbesondere dentales, Handstück (1), nach einem der vorstehenden Ansprüche, **dadurch gekennzeichnet, dass**
die elektrische Temperaturmessvorrichtung (12) über eine elektrische Leitung (22, 22', 22") mit einer Einheit (23) verbunden ist, die als Auswerteeinheit zum Auswerten der Messsignale der Temperaturmessvorrichtung (12) und / oder als Regel- oder Steuereinheit zum temperaturabhängigen Regeln oder Steuern des Betriebs des Handstücks (1) ausgebildet ist.

6. Medizinisches, insbesondere dentales, Handstück (1), nach einem der vorstehenden Ansprüche, **dadurch gekennzeichnet, dass**
eine in der Einheit (23) vorgesehene Strom- oder Spannungsquelle mit der elektrischen Leitung (22, 22', 22") und dem Halbleitersensor (27) oder dem metallischen Widerstand (26, 28) der elektrischen Temperaturmessvorrichtung (12) einen elektrischen Stromkreis bildet.

## Claims

1. A medical, in particular dental handpiece (1), comprising: a handle part (17) and a head part (16), in which a tool holder (15) is disposed for releasably connecting or accommodating a tool which can be connected to the handpiece (1), an outer case (2), wherein the head part (16) comprises a hollow head case (2') which forms an internal space (11), a drive device (3) for setting in motion a tool which can be connected to the handpiece (1), components which are moveable with respect to the head case (2') and which are accommodated in the head part (16), in particular bearings (5), the tool holder (15), at least portions of a transmission (6) or of gearwheels which transmit the drive movement of the drive device (3) to the tool holder (15), or at least portions of a device (7) for releasing the tool from the tool holder (15), and a temperature measuring device (4) for measuring the temperature of the handpiece (1) or a portion of the handpiece (1), wherein at least some of the components (5, 6, 7, 15) or portions thereof accommodated in the head part (16) and which are movable with respect to the head case (2') constitute heat sources which emit heat into the interior of the handpiece (1) and/or onto the head case (2'), wherein the temperature measuring device (4) is configured as an electrical temperature measuring device (12), wherein the electrical temperature measuring device (12) comprises a temperature sensor which is disposed in the head part (16) of the handpiece (1) and receives heat or thermal radiation from a plurality of the heat sources (5, 6, 7) disposed in the outer case or head case (2, 2') of the handpiece (1), wherein at least one of said plurality of heat sources comprises a movable component (5, 6, 7, 15), and measures an average temperature value or mean temperature value of the handpiece (1) or a portion of the handpiece (1) or an internal space (11) of the handpiece (1), which results from the emission of heat from the plurality of heat sources (5, 6, 7),
the electrical temperature measuring device (12) comprises a material the electrical resistance or dynamic resistance of which is variable as a function of the temperature, and
the temperature measuring device (12) is disposed between the heat sources (5, 6, 7).

2. The medical, in particular dental handpiece (1) according to claim 1, **characterized in that**
the temperature measuring device (12) in the internal space (11, 11') of the handpiece (1) is disposed at a distance from the heat sources (5, 6, 7) or **in that** the heat sources (5, 6, 7) surround the temperature measuring device (12) or **in that** the heat-emitting surfaces of the heat sources (5, 6, 7) are separated spatially from a surface of the temperature measuring device (12) receiving the heat from the heat sources (5, 6, 7), or **in that** the temperature measuring device (12) comprises a plurality of surfaces (8') receiving heat from the heat source (5, 6, 7) which in particular are orientated in different directions.

3. The medical, in particular dental handpiece (1) according to one of the preceding claims, **characterized in that**
the electrical temperature measuring device (12) comprises a semiconductor sensor (27), preferably a semiconductor diode, or a sensor with an ohmic resistance, an electrical, in particular metallic conductor, a ceramic, in particular a sintered metal oxide, or a doped semiconductor.

4. The medical, in particular dental handpiece (1) according to one of the preceding claims, **characterized in that**
the sensor is configured as an active sensor which requires a current or voltage supply in order to determine the temperature, or as a passive sensor, which does not require a current or voltage supply to determine the temperature.

5. The medical, in particular dental handpiece (1) according to one of the preceding claims, **characterized in that**
the electrical temperature measuring device (12) is connected via an electrical line (22, 22', 22") to a unit (23) which is configured as an evaluation unit in order to evaluate the measured signals from the temperature measuring device (12) and/or as a regulating or control unit for temperature-dependent regulation or control of the operation of the handpiece (1).

6. The medical, in particular dental handpiece (1) according to one of the preceding claims, **characterized in that**
a current or voltage source provided in the unit (23) forms an electrical circuit with the electrical line (22, 22', 22") and the semiconductor sensor (27) or the metallic resistance (26, 28) of the electrical temperature measuring device (12).

## Revendications

1. Pièce à main (1) médicale, en particulier dentaire, comprenant: une partie de poignée (17) et une partie de tête (16) dans laquelle est disposée un porte-outil (15) pour la liaison ou réception amovible d'un outil pouvant être relié à la pièce à main (1), un manchon externe (2), dans laquelle la partie de tête (16) présente un manchon de tête creux (2') formant un espace intérieur (11), un dispositif d'entraînement (3) pour mettre en mouvement un outil pouvant être relié à la pièce à main (1), des pièces mobiles par rapport au manchon de tête (2'), lesquelles sont réceptionnées dans la partie de tête (16), en particulier des paliers (5), le porte-outil (15), au moins des parties d'un engrenage (6) ou de roues dentées, lesquelles transmettent le mouvement d'entraînement du dispositif d'entraînement (3) au porte-outil (15), ou au moins des parties d'un dispositif (7) pour détacher l'outil du porte-outil (15), et un dispositif de mesure de température (4) pour mesurer la température de la pièce à main (1) ou d'une partie de la pièce à main (1), dans laquelle au moins certaines des pièces mobiles (5, 6, 7, 15) par rapport au manchon de tête (2') réceptionnées dans la partie de tête (6), ou au moins des parties de celles-ci, forment des sources de chaleur, lesquelles transmettent de la chaleur à l'intérieur de la pièce à main (1) et/ou au manchon de tête (2'), dans laquelle
le dispositif de mesure de température (4) est réalisé en tant que dispositif électrique de mesure de température (12), dans laquelle le dispositif électrique de mesure de température (12) présente un capteur de température qui est disposé dans la partie de tête (16) de la pièce à main (1) et qui réceptionne de la chaleur ou un rayonnement de chaleur de plusieurs sources de chaleur (5, 6, 7) disposées dans le manchon externe ou de tête (2, 2') de la pièce à main (1), dans laquelle au moins l'une de ces plusieurs sources de chaleur présente une pièce mobile (5, 6, 7, 15) et mesure une valeur à moyenne calculée de la température ou une valeur de température moyenne de la pièce à main (1) ou d'une partie de la pièce à main (1) ou d'un espace intérieur (11) de la pièce à main (1), laquelle résulte des émissions de chaleur des plusieurs sources de chaleur (5, 6, 7),
le dispositif électrique de mesure de température (12) comprenant un matériau dont la résistance électrique ou la résistance dynamique est variable en fonction de la température, et
le dispositif de mesure de température (12) étant disposé entre les sources de chaleur (5, 6, 7).

2. Pièce à main (1) médicale, en particulier dentaire selon la revendication 1, **caractérisée en ce que**
le dispositif de mesure de température (12) est disposé dans l'espace intérieur (11, 11') de la pièce à main (1) de manière espacée des sources de chaleur (5, 6, 7) ou bien **en ce que** les sources de chaleur (5, 6, 7) entourent le dispositif de mesure de température (12) ou bien **en ce que** les surfaces émettant de la chaleur des sources de chaleur (5, 6, 7) sont espacées spatialement d'une surface réceptionnant la chaleur de la source de chaleur (5, 6, 7) du dispositif de mesure de température (12) ou bien **en ce que** le dispositif de mesure de température (12) présente plusieurs surfaces (8') réceptionnant la chaleur de la source de chaleur (5, 6, 7), lesquelles sont en particulier orientées dans différentes directions.

3. Pièce à main (1) médicale, en particulier dentaire selon l'une des revendications précédentes, **caractérisée en ce que**
le dispositif électrique de mesure de température (12) présente un capteur à semiconducteur (27), de préférence une diode à semiconducteur, ou un capteur avec une résistance ohmique, un conducteur électrique, en particulier métallique, une céramique, en particulier un oxyde métallique fritté, ou un semiconducteur dopé.

4. Pièce à main (1) médicale, en particulier dentaire selon l'une des revendications précédentes, **caractérisée en ce que**
le capteur est réalisé en tant que capteur actif, lequel a besoin d'une alimentation en courant ou en tension pour la détermination de la température, ou en tant que capteur passif, lequel n'a pas besoin d'une alimentation en courant ou en tension pour la détermination de la température.

5. Pièce à main (1) médicale, en particulier dentaire selon l'une des revendications précédentes, **caractérisée en ce que**
le dispositif électrique de mesure de température (12) est relié via une ligne électrique (22, 22', 22") à une unité (23) qui est réalisée en tant qu'unité d'évaluation pour l'évaluation des signaux de mesure du dispositif de mesure de température (12) et/ou en tant qu'unité de réglage ou de commande pour le réglage ou la commande en fonction de la température du fonctionnement de la pièce à main (1).

6. Pièce à main (1) médicale, en particulier dentaire selon l'une des revendications précédentes, **caractérisée en ce que**
une source de courant ou de tension prévue dans l'unité (23) forme, avec la ligne électrique (22, 22', 22") et le capteur à semiconducteur (27) ou la résistance métallique (26, 28) du dispositif électrique de mesure de température (12), un circuit électrique.
